# EUROPEAN PATENT APPLICATION

(11) **EP 1 445 261 A1**
(43) Date of publication of application: **11.08.2004**
(21) Application number: 02775531.3
(22) Date of filing: 12.11.2002
(51) Int. Cl.: C07K 1/14, A23J 1/08, A23J 3/14, C12N 9/00

(54) **PROCESS FOR PRODUCING CONCENTRATED/PURIFIED PROTEIN USING CLAY MINERAL COMPOSITION**

(30) Priority: 13.11.2001 JP 2001347059
(71) Applicant: THE NISSHIN OIL MILLS, LTD., Tokyo, 104-8285 (JP)
(72) Inventor: MINOSHIMA, Ryoichi, c/o The Nisshin OilliO Ltd, Yokosuka-shi, Kanagawa 239-0832 (JP)
(74) Representative: Curtis, Philip Anthony
(86) International application number: PCT/JP2002/011768
(87) International publication number: WO 2003/042236

(57) **Abstract**

A method of separating a protein characterized in that the protein absorbed by a clay mineral composition is separated by using one or more members selected from a group consisting of fatty acid esters having 30 or less carbon atoms in the fatty acid residue. According to this method, a target protein can be conveniently and economically obtained in a large amount on an industrial scale at a high efficiency without deteriorating its functions (for example, lowering the enzymatic activity). Thus, the protein can be concentrated and purified.

## Description

### Technical Field

The present invention relates to, for instance, a method for preparing a concentrated, purified protein including, for instance, an enzyme protein or a physiologically active protein and a method for recovering such a protein.

### BACKGROUND ARTS

To isolate, concentrate and/or purify an aqueous solution containing a protein as an amphoteric electrolyte, there have conventionally and currently been used, for instance, ion-exchange techniques, gel filtration techniques, which make use of the molecular sieve effect, and isoelectric focusing techniques, which make use of the isoelectric point of protein. However, the isolation, concentrationand/orpurificationofacrudeprotein according to these conventional techniques require the use of various pre-treatments such as a preliminary desalting and/or concentration treatments of a protein-containing solution and therefore, these conventional techniques suffer from a variety of technical difficulties to be eliminated, when they are carried out in an industrial scale. For instance, they comprise quite complicated steps and this leads to an increase in the production cost of such a protein. For this reason, there has been desired for the development of a method, which permits the cost-saving and efficient isolation, concentration and/or purification of a protein.

It has been known for a long time that there are certain proteins capable of being specifically adsorbed on a clay mineral substance. In most of the cases, however, the protein is irreversibly adsorbed on the clay mineral substance and therefore, it is in general quite difficult to elute the protein once adsorbed on the mineral substance. Moreover, the elution of a protein once adsorbed on the substance requires any change of pH and/or ionic strength of an eluent, but such a treatment may be accompanied by any change in the higher-order structure of the protein and accordingly, there has not yet been developed any technique for simply and efficiently isolating, concentrating and/or purifying a protein through the elution using a cheap and easily available eluent.

There have conventionally been proposed some techniques for treating enzyme proteins while making use of a clay mineral.

For instance, Japanese Un-Examined Patent Publication No. Sho 51-70874 discloses a method for recovering and insolubilizing α-1,6-glucosidase characterized by the step of adsorbing α - 1,6-glucosidase on clay or diatomaceous earth. However, this patent simply discloses that the α - 1,6-glucosidase adsorbed thereon can be eluted by suspending the glucosidase in a common salt aqueous solution having a concentration ranging from 5 to 10% and then adjusting the pH value of the resulting mixture.

In addition, Japanese Un-Examined Patent Publication No. Sho 51-70873 discloses a method for adsorbing β -amylase derived from bacteria belonging to the genus Bacillus having an ability of producing β -amylase ( α -1,4-glucan malto-hydrolase) and α -1,6-glucosidase, the method comprising the step of adsorbing β -amylase derived from the bacteria on the starch, which has been heat-treated at a temperature ranging from 50 to 65°C, as well as a method for adsorbing β-amylase and α -1,6-glucosidase derived from bacteria belonging to the genus Bacillus on a mixture of the starch and diatomaceous earth, which has been heat-treated at a temperature ranging from 50 to 65°C. This patent discloses that β-amylase can be eluted with a maltose solution having a concentration ranging from 1 to 10%, usually 5 to 10% or a solution of partial hydrolyzates of starch such as a dextrin solution like starch syrup according to the former method and that the both enzymes can be eluted with a solution of partial hydrolyzates of starch (saccharide concentration: 1 to 10%, in general 5 to 10%) such as maltose containing common salt in an amount ranging from 5 to 10%.

As a recent proposal, Japanese Un-Examined Patent Publication No. Sho 63-132898 discloses a method comprising the steps of bringing an enzyme protein or a physiologically active protein into contact with a clay mineral and then eluting the protein with an aqueous solution of a polymeric compound as an eluent. However, this method suffers from a problem in that it is difficult to remove the polymeric compound brought into contact with the protein.

### DISCLOSURE OF THE INVENTION

An object of the present invention is to provide a technique for isolating, concentrating, purifying and/or recovering, in a high recovery, a highly purified protein adsorbable on a clay mineral-containing composition without impairing the higher- order structure of the protein responsible for the functionality thereof, under mild conditions using easy operations. It is another object of the present invention to provide a technique, which permits the concentration and/or purification of a protein in an industrial scale in a variety of fields such as medicines, enzymes, foods and wide variety of other fields, in which it has been quite difficult to concentrate and/or purify products in a large scale.

### Means for Solving the Problems

The inventors of this invention have conducted various studies to solve the foregoing problems, as a result, have found that proteins such as enzyme proteins or physiologically active proteins adsorbed on a clay mineral-containing composition can easily be isolated or separated from the composition through the use of a low molecular weight fatty acid ester whose fatty acid residue (or moiety) has a carbon atom number of not more than 30 and have thus completed the present invention.

More specifically, the present invention relates to a method for isolating a protein characterized in that a protein adsorbed on a clay mineral-containing composition is isolated using at least one member selected from the group consisting of fatty acid esters whose fatty acid residue has a carbon atom number of not more than 30. Preferably, the clay mineral-containing composition is at least one member selected from the group consisting of bentonite, montmorillonite, kaolinite, illite, chlorite, hydrotalcite, and burned and modified products thereof. Preferably, the at least one member selected from the group consisting of fatty acid esters, whose fatty acid residue has a carbon atom number of not more than 30, has an average molecular weight of less than 1000 and it comprises not less than 50% by mass of a fatty acid monoester on the basis of the total mass of the fatty acid ester. Preferably, the protein is at least one member selected from the group consisting of enzyme proteins and physiologically active proteins and it is also preferred that the protein is at least one member selected from the group consisting of lipid-related enzyme proteins. Moreover, the protein-isolation process is preferably conducted in the presence of moisture and it is more preferably carried out in an aqueous solution.

The present invention also relate to a method for preparing a concentrated and purified protein and the method comprises the following steps (I) and (II):
(I) A step for adsorbing a protein on a clay mineral-containing composition; and
(II) A step for isolating the protein adsorbed on the clay mineral-containing composition through the use of at least one member selected from the group consisting of fatty acid esters, whose fatty acid residue has a carbon atom number of not more than 30.

Preferably, the present invention relates to the foregoing method for preparing a concentrated and purified protein, wherein it further comprises the following steps (I-A) and/or (I-B) after the completion of the foregoing step (I):
(I-A) A step for recovering the clay mineral-containing composition on which the protein has been adsorbed;
(I-B) A step for purifying the clay mineral-containingcompositiononwhichtheprotein has been adsorbed.

Preferably, the present invention relates to the foregoing method for preparing a concentrated and purified protein, wherein the following step (III) is conducted after the completion of the foregoing step (II):
(III) A step for removing the fatty acid ester used for the isolation of the protein and/or a step for recovering the protein thus isolated.

Specifically, a concentrated and purified protein can be prepared by a method wherein a clay mineral-containing composition is added to a solution containing the protein and the resulting mixture is stirred to thus adsorb the protein on the composition; the clay mineral-containing composition carrying the protein adsorbed thereon is recovered by the use of a centrifuge or a membrane; and then the recovered clay mineral-containing composition carrying the protein adsorbed thereon is treated with at least one member selected from the group consisting of fatty acid esters, whose fatty acid residue has a carbon atom number of not more than 30, to thus isolate the protein. Alternatively, a concentrated and purified protein can be prepared by a method wherein a solution containing the protein is passed through a membrane and/or a column provided thereon with the clay mineral- containing composition to thus adsorb the protein on the mineral composition and then a solution of at least one member selected from the group consisting of fatty acid esters, whose fatty acid residue has a carbon atom number of not more than 30, is passed through the membrane and/or the column to thus isolate the protein.

In addition, the clay mineral-containing compositionusedhereinispreferablyonecomprising at least one member selected from the group consisting of bentonite, montmorillonite, kaolinite, illite, chlorite, hydrotalcite and burned and modified products thereof, the clay mineral-containing composition used for the foregoing adsorption has an average particle size ranging from 0.1 to 100µm. Preferably, the at least one member selected from the group consisting of fatty acid esters, whose fatty acid residue has a carbon atom number of not more than 30, has an average molecular weight of less than 1000 and it preferably comprises not less than 50% by mass of a fatty acid monoester on the basis of the total mass of the fatty acid ester. Preferably, the protein is at least one member selected from the group consisting of enzyme proteins and physiologically active proteins and it is also preferred that the protein is at least one member selected from the group consisting of lipid-related enzyme proteins. Moreover, the protein-isolation process is preferably conducted in the presence of moisture and it is more preferably carried out in an aqueous solution.

According to the present invention, any protein is concentrated and purified and the recovery of the protein is quite excellent and it is, for instance, not less than 70%. Therefore, the technique of the present invention is industrially preferred and is excellent in the production cost. Moreover, the electrical conductance (ms/cm) of the concentrated and purified protein thus obtained is not more than 5. Thismeans that the resulting protein has a very low content of impurities, which may adversely affect the activity of the protein and, for this reason, the protein has a high purity from the viewpoint of the activity. The protein is also excellent in the storage stability and function after the concentration and purification and is preferred as a product. In addition, the technique of the present invention is also preferred since it can concentrate and purify any protein to such an extent that the concentration rate as expressed in terms of the specific activity as an indication is not less than 3 times.

The present invention permits the preparation of a concentrated and purified protein composition according to the foregoing concentration and/or purificationmethod. Further, the storage stability of the resulting protein composition can be improved by the addition of, for instance, a water-soluble polymeric compound, divalent metal ions and/or an antioxidant. For instance, such a water-soluble polymeric compound is preferably a polyhydric compound among others and specific examples thereof are glucose, sucrose, glycerol, sorbitol, polyvinyl alcohol and polyethylene glycol. In addition, such divalent metal ions are, for instance, those of metals such as calcium, magnesium and copper. Moreover, such an antioxidant is, for instance, ascorbic acid, 2-mercapto- ethanol, cysteine and dithiothreitol. Examples of proteins to be treated according to the present invention are proteins contained in oilseeds such as soybean, rapeseeds and sesame seed and hydrolyzates of such oilseed proteins.

As has been described above, the protein is preferably at least one member selected from the group consisting of enzyme proteins and physiologically active proteins and preferably used herein also include at least one member selected from the group consisting of lipid-related enzyme proteins. These proteins are highly concentrated and purified or the content of impurities is considerably reduced as compared with that achieved prior to the treatment. For this reason and because of their improved functions, the concentrated and purified protein composition can be used in a variety of applications, for instance, they can be incorporated into, for instance, a variety of foods and beverages, medicines and cosmetics. More specifically, they can be used in the production steps of various foods and beverages and in the processes for preparing fats and oils and/or processed products thereof.

The present invention also relates to a protein-containing fatty acid ester composition obtained by separating proteins adsorbed on a clay mineral-containing composition using at least one member selected from the group consisting of fatty acid esters whose fatty acid residue has a carbon atom number of not more than 30. For instance, the protein-containing fatty acid ester composition can be obtained when separating the proteins from the clay mineral-containing composition in the foregoing concentration and/or purification treatments. If it is not necessary to remove the fatty acid ester from the resulting protein-containing fatty acid ester composition prior to the practical applications of the proteins, the fatty acid ester composition can be used without any pre-treatment. Preferably, the storage stability of the resulting protein- containing fatty acid ester composition can be improved by the additionof, for instance, awater-solublepolymeric compound, divalent metal ions and/or an antioxidant. For instance, such a water-soluble polymeric compound is preferably a polyhydric compound among others and specific examples thereof are glucose, sucrose, glycerol, sorbitol, polyvinyl alcohol and polyethylene glycol. In addition, such divalent metal ions are, for instance, those of metals such as calcium, magnesium and copper. Moreover, such an antioxidant is, for instance, ascorbic acid, 2-mercaptoethanol, cysteine and dithio- threitol. Examples of proteins are those contained in oilseeds such as soybean and rapeseeds, and sesame seed and hydrolyzates of such oilseed proteins. The fatty acid ester composition comprises at least one member selected from the group consisting of those listed above. Preferably, the protein is at least one member selected from the group consisting of enzyme proteins and physiologically active proteins, or at least one member selected from the group consisting of lipid-related enzyme proteins.

The present invention likewise relates to a method for recovering a protein comprising the following steps (I) and (II):
(I) A step for adsorbing a protein on a clay mineral-containing composition; and
(II) A step for isolating the protein adsorbed on the clay mineral-containing composition through the use of at least one member selected from the group consisting of fatty acid esters, whose fatty acid residue has a carbon atom number of not more than 30.

In particular, the present invention permits the recovery of enzyme proteins from processed liquids obtained during and/or after processes, which make use of enzyme reactions and waste liquor and therefore, the present invention is industrially favorable, in particular, in the processing cost.

The present invention also relates to a protein-isolation agent for isolating a protein adsorbed on a clay mineral-containing composition comprising at least one member selected from the group consisting of fatty acid esters, whose fatty acid residue has a carbon atom number of not more than 30. Preferably, the at least one member selected from the group consisting of fatty acid esters, whose fatty acid residue has a carbon atom number of not more than 30, has an average molecular weight of less than 1000 and it is also preferred that the fatty acid ester comprises not less than 50% by mass of a fatty acid monoester on the basis of the total mass of the fatty acid ester.

### [ Best Mode for Carrying Out the Invention]

The present invention mainly relates to a method for isolating or separating proteins adsorbed on a clay mineral-containing composition using a fatty acid ester whose fatty acid residue has a carbon atom number of not more than 30 and has completed, for instance, a method for concentrating and purifying proteins as well as a method for recovering the proteins, while applying the foregoing isolation method.

It has been known that a protein is adsorbed on a clay mineral and there have been known techniques for separating the protein from the clay mineral in which the protein adsorbed on the clay mineral is isolated through the use of an eluent having a different pH value or an ionic strength, but these techniques are accompanied with an abrupt change of pH or ionic strength, which may cause various changes in the higher-order structure of the protein strictly involved in the functionality thereof. Moreover, they require post-treatments of the eluted protein such as dialysis for the deionization of the protein and therefore, these techniques suffer from a problem in that processes for handling a large quantity of proteins in an industrial scale are quite complicated and quite expensive. Alternatively, there have also been known methods wherein a polymeric compound is added to a system containing a protein to be isolated and according to these methods, deionized, concentrated and purified protein can be isolated by the use of the polymeric compound as an eluent. However, they suffer from a problem such that the protein is never eluted if a polymeric compound solution having a relatively high concentration is added and the resulting mixture is allowed to stand over not less than 30 minutes with stirring. In addition, the eluted protein aqueous solution containing the added polymeric compound has a relatively high viscosity and this would adversely affect the activity of the protein in the subsequent post-treatments such as the granulation or powdering of the protein. Moreover, the polymeric compound in general has a high molecular weight comparable to that of the protein, it is thus difficult to fractionate the same through the usual concentration and/or purification techniques such as the ultrafiltration and gel filtration techniques and therefore, it is necessary to adopt adsorption treatments such as the ion-exchange technique. In this ion-exchange technique, the protein should be eluted using an eluent having a high ionic strength and this in turn requires the use of a deionization treatment such as dialysis as a post-treatment, but the processes required for handling a large quantity of such a protein in an industrial scale are quite complicated and quite expensive. Moreover, in the technique using such a polymeric compound as an eluent, the recovery of the eluted protein is, at present, simply not more than 50%, even if such industrial, operational and economical problems are solved to a considerable extent.

On the other hand, the inventors of this invention have found that the adsorbed protein can easily be isolated using a fatty acid ester whose fatty acid residue has a carbon atom number of not more than 30 and that a protein solution having a high concentration can be obtained. For instance, the addition of a fatty acid ester whose fatty acid residue has a carbon atom number of not more than 30 permits the easy separation of the adsorbed protein without stirring and allowing the mixture to stand and thus the protein can be recovered at a high recovery on the order of, for instance, not less than 70%, preferably not less than 75%, further preferably not less than 80%, more preferably not less than 85%, particularly preferably not less than 90% and most preferably not less than 95%.

In addition, the isolation method according to the present invention easily and cheaply permits the bulk handling in an industrial scale and is also industrially and economically excellent.

In the present invention, the clay mineral composition used for adsorbing proteins thereon means a composition comprising a clay mineral and may be those subjected to a variety of modification treatments. Moreover, the clay mineral herein used means substances containing a group of water-containing silicates, which impart plasticity to clay. The clay mineral-containing composition used herein is preferably those only comprising clay minerals among others.

In the present invention, any known clay minerals can be used and specific examples thereof are bentonite, montmorillonite, kaolinite, illite, chlorite, hydrotalcite, and burned and modified products thereof. These clay minerals can be used, in the present invention, alone or in any combination of at least two thereof. Preferred examples thereof include, but are not limited to at least one member selected from the group consisting of bentonite, montmorillonite, kaolinite and burned and modified products thereof, with bentonite and burned and modified products thereof being further preferably used herein.

The shape and the amount of the clay mineral-containing composition to be used may appropriately be selected. For instance, examples of shapes thereof include powdery, particulate, granular, pellet-like, massive shapes. In addition, it is sufficient to use the clay mineral-containing composition in an amount ranging from 0.1 to 50 times, preferably 0.5 to 20 times and more preferably 1 to 5 times the mass of the protein adsorbed on and fixed to the composition.

Other components can likewise be incorporated into the composition for the purpose of imparting various functions such as contact properties, water dispersibility and sedimentation properties as well as shape-retention ability.

Examples of such components or additives include, but are not limited to, diatomaceous earth, silica sand and cellulose fibers.

The foregoing modification treatment may be, for instance, organicmodificationtreatments. More specifically, examples of such modified products include layer surface-modified bentonite treated with quaternary ammonium or anionic polymers; and layer surface-modified, multi-modified or edge face-modified bentonite treated with alkyl trialkoxy silane, carboxyvinyl polymers, propylene carbonate and combinations of quaternary ammonium + alkyl trialkoxy silanes. The composition may be converted into a hydrophobic one by the modification withquaternaryammoniumandalkyltrialkoxysilanes and the resulting hydrophobic composition can be used in organic solvents and plastics. High dispersibility and/or low viscous properties can be imparted to the composition by the modification with an anionic polymer, while highly viscous properties can be imparted to the composition by the modification with carboxyvinyl polymers and propylene carbonate.

With respect to the adsorption of a protein on the clay mineral-containing composition, the surface of the composition is negatively charged and therefore, the composition can electrically adsorb a positively charged substance. A particular protein has an isoelectric point peculiar thereto, can positively charged when the pH value of the protein solution is higher than the isoelectric point and therefore, the protein is electrically adsorbed on the composition, for instance, bentonite. In this respect, the hydrophobic amino acids present in the protein may be linked to bentonite through the hydrophobic bonding with silica (Si) as a constituent ofmontmorillonite, which is a principal component of the bentonite.

Specific examples of proteins capable of being adsorbed on the clay mineral-containingcomposition used in the present invention include simple proteins, which only comprise amino acids and composite proteins containing constituents other than amino acids. Examples of simple proteins include, but are not limited to albumin, globulin, prolamin, glutelin, histone, protamine and scleroprotein. Examples of composite proteins include, but are not limited to nucleoproteins, glycoproteins, lipoproteins, chromoproteins and metalloproteins. Examples thereof also include functional proteins such as physiologically active proteins and enzyme proteins.

Specific examples of physiologically active proteins are proteins derived from, for instance, soybean, wheat and sesame seed such as soybean globulin, wheat globulin, and wheat albumin and sesame globulin; blood proteins such as serum albumin and hemoglobin; milk proteins such as lactoalbumin and lactoglobulin; and egg proteins such as ovoglobulin.

The protein can maintain its higher-order structure under the conditions for the isolation according to the present invention and therefore, the present invention is preferred since it permits the isolation of any physiologically active protein while maintaining the original functions thereof.

The enzyme protein may, for instance, be oxidreductase, a transferase (transfer enzyme), a hydrolase, a lyase (elimination enzyme), an isomerase (isomerization enzyme) and a ligase (synthase). Specific examples of enzyme proteins are lipase, phospholipase, esterase, protease, amylase, isomerase, oxygenase, lysozyme, urokinase and asparaginase. The enzyme protein never loses its activity under the conditions for the isolation according to the present invention and therefore, the present invention is preferred since it permits the isolation of any enzyme protein while maintaining the original functions thereof.

The enzyme protein is more preferably at least one member selected from the group consisting of lipid-related enzyme proteins.

Examples of lipid-related enzyme proteins are lipase, phospholipase and esterase. Preferred examples thereof are triacyl glycerol lipase, diacyl glycerol lipase, monoacyl glycerol lipase and phospholipases A1, A2, B, C and D.

The lipid-related enzyme protein never loses its activity under the conditions for the isolation according to the present invention and therefore, the present invention is preferred since it permits the isolation of any lipid-related enzyme protein while maintaining the original functions thereof.

In the present invention, proteins are separated from a clay mineral-containing composition on which the proteins are adsorbed using at least one member selected from the group consisting of fatty acid esters whose fatty acid residue has a carbon atom number of not more than 30. Examples of such fatty acid esters whose fatty acid residue has a carbon atom number of not more than 30 are fatty acid monoesters, fatty acid di-esters, fatty acid tri-esters and fatty acid poly-esters, all of which may preferably be used in the present invention.

In this respect, when separating the protein adsorbedontheclaymineral-containingcomposition and the clay mineral-containing composition provided thereon with the protein adsorbed is present in an oil, the fatty acid ester whose fatty acid residue has a carbon atom number of not more than 30 has an HLB value ranging from 0 to 5, preferably 0 to 4 and more preferably 2 to 3, while if the composition is present in an aqueous solution, the protein can efficiently be separated from the clay mineral- containing composition using a fatty acid ester having an HLB value of not less than 5, preferably 10 to 20 and more preferably 15 to 18.

These requirements for the HLB value and the molecular weight of the fatty acid ester whose fatty acid residue has a carbon atom number of not more than 30 can be satisfied by the use of a single fatty acid ester, but the HLB value of the fatty acid ester can preferably be controlled to a desired level through the use of two kinds of fatty acid esters.

Examples of such fatty acid esters whose fatty acid moiety has a carbon atom number of not more than 30 are sorbitan fatty acid esters, sucrose fatty acid esters, glycerin fatty acid esters, polyglycerin fatty acid esters, lecithin and enzyme-decomposed lecithin.

Specific examples of sorbitan fatty acid esters are sorbitan trioleate (HLB 1.8), sorbitan tristearate (HLB 2.1), sorbitan distearate (HLB 4.4), sorbitan mono-stearate (HLB 4.7), sorbitan mono-palmitate (HLB 6.7), sorbitan mono-oleate (HLB 4.3) and sorbitan mono-laurate (HLB 8.6).

Specific examples of sucrose fatty acid esters are sucrose stearic acid monoester (HLB 16) , sucrose oleic acid trimester (HLB 1), sucrose myristic acid diester (HLB 11), sucrose behenic acid diester (HLB 3), sucrose oleic acid monoester (HLB 15) and sucrose erucic acid triester (HLB 2).

Specific examples of glycerin fatty acid esters are glycerol monostearate and glycerol monooleate. Specific examples of polyglycerin fatty acid esters are decaglycerin lauric acid ester, decaglycerin stearic acid ester and decaglycerin palmitic acid ester.

Moreover, specific examples of lecithin and enzyme-decomposed lecithins are phosphatidyl choline, phosphatidyl serine, phosphatidyl ethanolamine, phosphatidyl inositol, phosphatidic acid and lyso-isomers thereof or mixture thereof.

When the protein is isolated in an oil solution, it is preferred to use, for instance, sucrose fatty acid esters, glycerin fatty acid monoesters, sorbitan fatty acid esters, sorbitan fatty acid polyesters and propylene glycol fatty acid esters, but the fatty acid esters used in this case are not restricted to these specific ones at all. On the other hand, when the protein is separated in an aqueous solution, it is preferred to use sucrose fatty acid esters and sorbitan fatty acid esters, but the fatty acid esters used in this case are not likewise restricted to these specific ones.

The at least one fatty acid ester whose fatty acid moiety has a carbon atom number of not more than 30 preferably has an average molecular weight of less than 1000, more preferably less than 900, more preferably less than 800, more preferably less than 700, more preferably less than 600, more preferably less than 550 and most preferably less than 530, but the fatty acid ester is not restricted to those listed above. This is because, the smaller the molecular weight of the fatty acid ester, the easier the concentration and purification of proteins and the easier the removal of the fatty acid ester.

As the at least one member selected from the group consisting of fatty acid esters whose fatty acid moiety has a carbon atom number of not more than 30, it is preferred to use those having a content of fatty acid monoesters of not less than 50% by mass on the basis of the total mass of the fatty acid esters since the amount thereof to be removed becomes small. Since the smaller the molecular weight of the fatty acid ester, the easier the separation of proteins and the removal of the fatty acid ester, it is particularly preferred to use fatty acid monoesters and the content of the fatty acid monoester is thus not less than 50% by mass, preferably not less than 60% bymass, more preferably not less than 70% by mass and particularly preferably not less than 80% by mass.

From the same reason, the number of carbon atoms constituting the fatty acid moiety of the fatty acid ester is preferably small and it is not more than 30, it is preferably not more than 24, more preferably not more than 18 and most preferably not more than 14.

When practically using these fatty acid esters for the isolation of proteins, the amount of such a fatty acid ester whose fatty acid moiety has a carbon atom number of not more than 30 to be used is not restricted to any particular range, but the higher the concentration of the fatty acid ester, the higher the isolation efficiency achieved. The content thereof ranges from 0.001 to 10% by mass, preferably 0.01 to 5.0% by mass, more preferably 0.05 to 1.0% by mass from the viewpoint of, for instance handling properties, but the present invention is not restricted to the foregoing specific range.

In the invention, the adsorbed protein is eluted by dispersing a clay mineral-containing composition on which the protein is adsorbed in a solution of a fatty aid ester.

If the amount of the fatty acid ester relative to the protein is controlled to the ratio ranging from 1: 0.001 to 20, preferably 1: 0.01 to 10 and more preferably 1: 0.05 to 2, preferred conditions such as the separation ability and usability of the resulting protein can be established.

According to an embodiment of the foregoing isolation, if the isolation is, for instance, carried out in an aqueous solution, the fatty acid ester whose fatty acid moiety has a carbon atom number of not more than 30 and which has an HLB value preferably ranging from 5 to 20, more preferably 10 to 20 and most preferably 15 to 18 is added in an amount relative to the protein to be isolated ranging from 1: 0.001 to 20, preferably 1: 0.01 to 10 and more preferably 1: 0.05 to 2 (or the fatty acid ester is added in a low concentration on the order of not more than 1% by mass per unit mass of an enzyme solution) to thus concentrate the protein adsorbed on a clay mineral-containing composition to a high concentration on the order of not less than 95% without stirring and allowing to stand. In this respect, the mixture may be stirred at a rate preferably ranging from 5 to 500 rpm, more preferably 10 to 300 rpm and most preferably 50 to 200 rpm using a stirring machine (or ultrasonics may likewise be applied to the mixture). Other conditions such as temperature and pressure are not restricted to particular ranges, but the processing temperature preferably ranges from 0 to 10°C and the pressure may be ordinary pressure. Thereafter, the clay mineral-containing composition is removed to thus recover the concentrated and purified protein. Moreover, the fatty acid ester having a carbon atom number of not more than 30 and the excess of the solution may be removed to isolate and recover the protein at an extremely high concentration. The isolation system may be either an aqueous system or an oil system, but the aqueous system is particularly preferred while taking into consideration, for instance, the workability, the properties of proteins to be isolated and the preparation of enzymes through cultivation.

Examples of methods for removing the clay mineral-containing composition include, but are not restricted to, the separation by allowing the processed system to stand (decantation) and the centrifugation. Examples of centrifugation methods include, but are not restricted to, those using decanter type centrifugal machines, bucket type centrifugal machines and separation plate type centrifugal machines.

The method for removing the fatty acid ester whose fatty acid moiety has a carbon number of not more than 30 is not restricted to any particular one, but the fatty acid ester can easily be removed by the gel filtration method or a method using an ultrafiltration membrane, while making use of the difference in molecular weight between the clay mineral and the protein.

As a specific method, the clay mineral-containing composition after the adsorption of proteins is removed by the precipitation, filtration and/or centrifugation, followed by optionally washing the same several times with water or a buffer solution to thus remove the remaining unadsorbed substances and then the adsorbed proteins can be isolated from the composition using, for instance, an aqueous solution of the foregoing fatty acid ester whose fatty acid moiety has a carbon atom number of not more than 30. The fatty acid ester whose fatty acid moiety has a carbon atom number of not more than 30 is added to water or deionized water in an amount ranging from 0.1 to 10% by mass and preferably 0.1 to 3% by mass on the basis of the water or deionized water used followed by dissolution thereof in water at room temperature or by heating to a temperature ranging from 30 to 50°C and then cooling the resulting solution to prepare an aqueous solution of the fatty acid ester. The protein can be isolated simply by adding and dispersing the clay mineral-containing composition carrying the protein adsorbed thereon in the resulting aqueous solution. Subsequently, the clay mineral-containing composition can easily be removed by the centrifugation, filtration and/or decantation. If the fatty acid ester whose fatty acid moiety has a carbon atom number of not more than 30 is removed from the protein thus isolated, the former can easily be removed by the gel filtration method or a method using an ultrafiltration membrane, while making use of the difference in molecular weight between the clay mineral and the protein. Moreover, the process for isolating the protein can be conducted at room temperature and therefore, it is not necessary to particularly cool or heat the processing system. However, the system may be heated to a temperature ranging from, for instance, about 0 to about 60°C.

These operations and conditions can be used in, for instance, the concentration and/or purification method as will be described below.

As will also be described below, if the clay mineral-containing composition is recovered using a membrane, the recovered clay mineral-containing composition or that still adhered to the membrane is subjected to, for instance, washing and then the fatty acid ester whose fatty acid moiety has a carbon atom number of not more than 30 is passed through the membrane carrying the composition adhered thereto to thus easily isolate the protein adsorbed on the composition and to thus give concentrated and/or purified proteins.

When using a clay mineral-containing composition in a solid state, the solid clay mineral is brought into contact with a starting liquid containing proteins to thus adsorb the proteins on the clay mineral, the clay mineral is subjected to, for instance, washing without any intermediate treatment and then the fatty acid ester whose fatty acid moiety has a carbon atom number of not more than 30 is passed through the solid clay mineral layer to thus easily isolate the proteins. In other words, it is sufficient to simply fix the solid clay mineral during the isolation process and therefore, this technique can be used in industrial production processes like the foregoing method.

A protein adsorbed on a clay mineral-containing composition has conventionally been eluted with a polymeric compound or a salt. For instance, the clay mineral- containing composition carrying the protein adsorbed thereon is added to and dispersed in a polyvinyl alcohol solution to thus elute the protein. Then the system is subjected to a separation treatment using an ion-exchange resin to remove the polyvinyl alcohol. The pH value of the enzyme solution is adjusted so that it falls within the neutral region to adsorb the protein on the ion-exchange resin and to thus remove or elute only the polyvinyl alcohol. The protein adsorbed on the ion-exchange resin is eluted by immersing the resin in a 0.5M sodium chloride aqueous solution. Subsequently, it is necessary to treat the protein-containing aqueous solution with the ultrafiltration membrane or the gel filtration column to removed the salt included in the aqueous solution. When the protein-containing aqueous solution is treated using an ultrafiltration membrane, a membrane of hollow fibers made of a material (polyacrylonitrile) whose molecular weight cutoff is on the order of about 6, 000 or about 13, 000 is operated at an inlet pressure and a delivery pressure set at 0.05 MPa and 0.12 MPa, respectively and a flow rate of the aqueous solution containing the protein maintained at 20 L/m²·hr to thus concentrate the solution to a volume of about 1/10 time the original one, followed by addition of water in an amount of 20 times the volume of the concentrate, concentration of the diluted solution to a volume identical to that prior to the dilution to thus ensure the desalting of the solution. Alternatively, if the desalting treatment is carried out according to the gel filtration, the protein-containing solution is loaded on a column packed with Sephadex G-200 at a flow rate of 0.3 mL/hr/mL per unit volume (1 mL) of the column while passing water through the column in an amount of not less than 5 times the volume of the protein solution to thus fractionate only the protein.

According to the conventional methods, the protein adsorbed on the clay mineral-containing composition is purified by the foregoing procedures and then the purified protein must be subjected to a treatment for the removal of the polymeric compound and a desalting treatment. In other words, the conventional methods are quite complicated and expensive and therefore, they are not practical, while the present invention permits the quite easy separation of the protein through the use of, for instance, the following agents for separation and does not require the use of the foregoing complicated post-treatments.

Further the present invention also relates to a protein-isolation agent for isolating a protein adsorbed on a clay mineral-containing composition comprising at least one member selected from the group consisting of fatty acid esters, whose fatty acid moiety has a carbon atom number of not more than 30.

The use of the protein-isolation agent permits the favorable separation of a protein adsorbed on a clay mineral-containing composition and the agent is suitably used in the methods for isolating, concentrating and/or purifying and recovering proteins according to the present invention. The fatty acid ester whose fatty acid moiety has a carbon atom number of not more than 30 has already been described above in detail and preferably used are fatty acid esters whose fatty acidmoiety has a carbon atom number of not more than 30 and which have HLB values corresponding to aqueous solutions and oil solutions. Moreover, the concentration thereof can be controlled depending on the intended purposes such as the foregoing isolation, concentration/purification and recovery of proteins and it is also possible to incorporate other components into the protein-isolation agent.

The protein-isolation agent comprises a fatty acid ester whose fatty acid moiety has a carbon atom number of not more than 30, but the content of the latter in the isolation agent is not restricted to any particular range and the higher the concentration of the fatty acid ester, the higher the protein-isolation ability of the agent. The content of the fatty acid ester in the isolation agent ranges from 0.01 to 50% by mass, preferably 0.5 to 30% by mass, more preferably 0.1 to 5% by mass, particularly preferably 0.1 to 2.0% by mass and most preferably 0.5 to 2.0% by mass in the light of, for instance, the handling properties thereof, but the invention is not restricted to the foregoing specific range.

Moreover, the suitable conditions such as the ability of protein-separation and usability can be achieved by adjusting the ratio thereof relative to the protein to be isolated to the range of from 1: 0.001 to 20, preferably 1: 0.01 to 10 and more preferably 1: 0.05 to 2, although the ratio is determined while taking into consideration the amount of the agent used.

The storage stability of the protein-isolation agent may be improved by incorporating additives such as a water-soluble polymeric compound, divalent metal ions and/or an antioxidant into the agent. For instance, such a water-soluble polymeric compound is preferably a polyhydric compound among others and specific examples thereof are glucose, sucrose, glycerol, sorbitol, polyvinyl alcohol and polyethylene glycol. In addition, such divalent metal ions are, for instance, those of metals such as calcium, magnesium and copper. Moreover, such an antioxidant is, for instance, ascorbic acid, 2-mercaptoethanol, cysteine and dithiothreitol. Examples of proteins capable of being isolated using the agent are those contained in oilseeds such as soybean, rapeseeds and sesame seed and hydrolyzates of such oilseed proteins.

The additives in liquid states at ordinary temperature are added to oil without any pretreatment and dissolved therein with stirring the mixture at a rate preferably ranging from 50 to 500 rpm, more preferably 100 to 300 rpm and most preferably 100 to 200 rpm using a stirrer or a three-one motor equipped with a stirring blade. Moreover, in case of additives in powdery and solid states and those in hardly soluble liquid states, at ordinary temperature, oil is heated to a temperature ranging from 40 to 50°C and then these additives are added thereto to give an oil solution.

Alternatively, the additives in liquid states at ordinary temperature are added to water without anypretreatment and dissolved therein with stirring the mixture at a rate preferably ranging from 50 to 500 rpm, more preferably 100 to 300 rpm and most preferably 100 to 200 rpm using a stirrer or a three-one motor equipped with a stirring blade. Moreover, in case of additives in powdery and solid states and those in hardly soluble liquid states, at ordinary temperature, it is preferably dissolved in water by heating the water in advance to a temperature ranging from 40 to 50°C and then adding these additives to the warmed water to give an aqueous solution.

The protein-isolation agent according to the present invention is quite favorable from the viewpoint of handling properties since the fatty acid ester having a carbon atom number of not more than 30 is in a liquid state and has a low viscosity.

The present invention provides a method for concentrating and/or purifying proteins while making use of the foregoing protein-isolation agent. More specifically, the method for concentrating and purifying a protein comprises the following steps (I) and (II):
(I) A step for adsorbing a protein on a clay mineral-containing composition; and
(II) A step for isolating the protein adsorbed on the clay mineral-containing composition through the use of at least one member selected from the group consisting of fatty acid esters, whose fatty acid residue has a carbon atom number of not more than 30.

In other words, proteins included in, for instance, a solution is adsorbed on a clay mineral-containing composition and then isolated using a fatty acid ester whose fatty acid moiety has a carbon atom number of not more than 30 to thus give concentrated and/or purified proteins.

In respect of the treatments used in the present invention, the methodology, places where the method is carried out and other conditions are not restricted to particular ones inasmuch as they can achieve the intended purposes. In the production method or the like of the present invention, only the procedures for production are specified and the term "procedures" herein used of course includes so-called production processes, but the term is not restricted thereto. In particular, every processing steps are not necessarily present in different places, these steps are not necessarily carried out in different machinery and tools, the intervals between different steps are not limited to any specific one and these steps may be conducted continuously or at constant intervals. In other words, these steps are not limited in place, facilities and time, at all.

Further, this method further comprises the following steps (I-A) and/or (I-B) after the completion of the foregoing step (I) in order to obtain a protein highly concentrated, highly purified through the exhaustive removal of impurities and accordingly, highly purified:
(I-A) A step for recovering the clay mineral-containingcompositiononwhichtheprotein has been adsorbed;
(I-B) A step for purifying the clay mineral-containingcompositiononwhichtheprotein has been adsorbed.

In this respect, these steps (I-A) and (I-B) may be carried out in any order.

In the method, the following step (III) is conducted after the completion of the foregoing step (II) to thus actually obtain a concentrated and/or purified protein:
(III) A step for removing the fatty acid ester used for the isolation of the protein and/or a step for recovering the protein thus isolated.

The resulting concentrated and/or purified protein may further be subjected to various treatments such as removal of impurities according to the usual methods.

As the foregoing concentration and/or purification method, there may be listed, for instance, one comprising the steps of adding a clay mineral-containing composition to a solution containing a protein with stirring to thus adsorb the protein on the composition, recovering the clay mineral-containing composition on which the protein is adsorbed using a centrifugal machine or a membrane and then separating the protein from the recovered composition carrying the protein adsorbed thereon using at least one member selected from the group consisting of fatty acid esters whose fatty acid moiety has a carbon atom number of not more than 30.

Alternatively, another example of such a method comprises the steps of passing a protein-containing solution through a membrane and/or a column to which a clay mineral-containing composition is fixed to thus adsorb the protein on the mineral composition and then passing a solution containing at least one member selected from the group consisting of fatty acid esters whose fatty acid moiety has a carbon atom number of not more than 30 through the membrane and/or column to thus isolate the protein. As a specific example, a clay mineral-containing composition is added simultaneously or separately with diatomaceous earth and/or fibrous cellulose to pre-coat a filter press and then a solution of a protein is passed through the filter press at a pressure of 0.1 MPa. The diatomaceous earth and fibrous cellulose are used in an amount preferably ranging from 0.5 to 50 times, more preferably 0.5 to 10.0 times and most preferably 1 to 4 times the mass of the protein to be isolated. After the adsorption of the protein on the composition, the composition carrying the protein adsorbed thereon is washed with water in an amount of 1 to 10 times the volume of the protein solution while applying a pressure ranging from 0.01 to 0.2 MPa to thus wash out the unadsorbed substance. Thereafter, a 0.01 to 10.0% fatty acid ester-containing solution as a protein-isolation agent is passed through the membrane and/or column in an amount of 3 times the volume of the protein-containing solution under a pressure ranging from 0.01 to 0.5 MPa to thus isolate and recover the intended protein.

Other methods and processing conditions can easily be determined or selected in the light of the description of the foregoing isolation method and various conditions as will be detailed below.

As raw materials containing proteins to be concentrated and/or purified, there may be listed, for instance, protein solutions. Examples of such protein solutions are protein solutions obtained in the course of or during the protein production processes, and enzyme protein-containing solutions used for the manufacture of other foods as well as solutions containing various proteins derived from cells and body fluids of animals, cells and seeds of plants, and fermentation products of microorganisms.

A method for adsorbing the protein present in a raw material such as the foregoing protein solution onaclaymineral-containingcompositioncomprises, for instance, the steps of adding the clay mineral-containing composition to the protein solution and then stirring the resulting mixture by the stirring with a stirrer, a propeller, treating in a homogenizer or the application of ultrasonics. For instance, the mixture is stirred with a stirrer or a three-one motor equipped with a stirring blade at a rate preferably ranges from 50 to 500 rpm, more preferably 100 to 300 rpm and most preferably 100 to 200 rpm. The stirring time preferably ranges from one minute to 5 hours, more preferably one minute to one hour and most preferably 1 to 30 minutes. When using a homogenizer as a stirring means, the rotational frequency thereof preferably ranges from 10 to 6000 rpm, more preferably 50 to 500 rpm and most preferably 50 to 300 rpm and the processing time preferably ranges from one minute to one hour, more preferably 1 to 30 minutes and most preferably 1 to 15 minutes.

Alternatively, the clay mineral-containing composition is fixed to a membrane or a column in advance and then a protein solution is passed through the membrane or column carrying the composition fixed thereto to thus adsorb the protein on the composition.

Examples of methods for recovering the clay mineral-containing composition carrying the protein adsorbed thereon include the centrifugation techniques, sedimentation techniques, filtration techniques, which make use of membranes or techniques for collecting the same through chromatography.

In addition, examples of methods for separating and removing the clay mineral-containing composition from which the protein has been removed are methods for allowing the reaction system to stand (decantation technique), centrifugation techniques and separation techniques using membranes, but the present invention is not restricted to these specific techniques. The method of leaving at rest comprises leaving the reaction system at rest over not less than 30 minutes and then removing the resulting supernatant through decantation using a siphon to thus concentrate and separate the clay mineral-containing composition. Examples of centrifugal machines usable herein include, but are not limited to, a decanter type centrifugal machine, a bucket type centrifugal machine and a separation plate type centrifugal machine. Regarding the conditions for the centrifugation, the rotational frequency of the machine preferably ranges from 300 to 10,000 rpm, more preferably 300 to 3,000 rpm and most preferably 600 to 2,000 rpm and the centrifugation time preferably ranges from one minute to one hour, more preferably 1 to 30 minutes and most preferably 5 to 15 minutes. Moreover, in the separation technique using a membrane, a filter press and an unglazed filtering device may be used and they may be pre-coated with diatomaceous earth or fibrous cellulose prior to the practical use. The particle size of the diatomaceous earth preferably ranges from 0.01 to 100 µm, more preferably 0.05 to 50 µm and most preferably 0.1 to 10 µm. Such a separation method is quite preferred since the method is excellent in the ability of coming to contact with proteins and can suitably adsorb the proteins thereon.

The conditions and the like for separating the protein from the clay mineral-containing composition are the same as those already described above.

The present invention relates to a protein-containing composition of a fatty acid ester whose fatty acid moiety has a carbon atom number of not more than 30, which is obtained by the method for separating, concentrating/purifying and recovering the protein. It is a key point of a series of the inventions that a protein adsorbed on a clay mineral-containing composition is separated using a fatty acid ester whose fatty acid moiety has a carbon atom number of not more than 30. In this respect, the fatty acid ester obtained after the separation is in the form of a composition containing the protein in a high concentration. This composition can be used in, for instance, foods and beverages and medicines without any post-treatment and can further be used in a variety of manufacturing processes.

The protein composition containing the fatty acid ester, as such, may be used in, for instance, fats and oils, foods and beverages and medicines. Moreover, the protein composition can be dispersed in fats and oils, preferably a middle chain fatty acid triglyceride in an amount preferably ranging from 1 to 20 times, more preferably 1 to 10 times and most preferably 2 to 5 times the mass of the protein, followed by recovery through filtration to thus separate the fatty acid ester and to use the resulting product. The filtration may be aspiration filtration through a Buchner funnel provided with filter paper, and filtration through, for instance, a Buchner funnel pre-coated with diatomaceous earth or a filter press.

Preferably, the foregoing composition comprises at least one member selected from the group consisting of isolated enzyme proteins and physiologically active proteins or comprises at least one member selected from the group consisting of lipid-related enzyme proteins and in a preferred embodiment, the composition in this state can directly be used or incorporated into a variety of goods.

As has been described above, the protein composition can be used in the production of, for instance, foods and medicines while containing the fatty acid ester whose fatty acid moiety has a carbon atom number of not more than 30 and can be directly incorporated into these goods. When it is intended to obtain a highly concentrated and highly purified protein composition, it is necessary to remove the fatty acid ester whose fatty acid moiety has a carbon atom number of not more than 30, which is added for the separation and the fatty acid ester can easily be removed according to the foregoing method. In this respect, the fatty acid ester having a low molecular weight is preferably used, in the light of the subsequent removal of the fatty acid ester whose fatty acid moiety has a carbon atom number of not more than 30.

In general, the concentration of a protein is carried out by, for instance, the ultrafiltration techniques, salting out techniques and solvent-precipitation techniques, but the membrane used in the ultrafiltration is quite expensive and this leads to an increase in the processing cost and the precipitation technique using ammonium sulfate or ammonium chloride requires the use of a subsequent desalting treatment and this makes the processing complicated. Moreover, the precipitation technique using a solvent such as acetone, hexane or alcohol requires the use of a large-scale device for the recovery of the solvent.

Moreover, the purification of a protein is in general carried out by the column chromatography. Examples of column chromatography techniques usable herein are ion-exchange, gel filtration and affinity chromatography techniques. In the ion- exchange chromatography technique, the protein adsorbed on an ion-exchange resin is released using a salt such as NaCl and therefore, this technique requires the use of a desalting treatment after the chromatography procedures. In the affinity chromatography technique, the carrier to be used is quite expensive and the addition of a salt is likewise necessary for the release of the adsorbed protein. The gel filtration technique does not require the use of any separation agent, but the carrier to be used is likewise very expensive.

According to the method of the present invention, a protein is concentrated and purified through the adsorption of the protein on a carrier and the desorption thereof from the carrier. For this reason, the method of the invention can be carried out using a carrier, which is cheap as compared with, for instance ion-exchange resins and the carrier can be removed using existing facilities such as a centrifugal machine. In addition, the method does not require the use of any complicated installation such as columns, the separation agent added in the post-treatment can easily be removed while making use of the difference in the molecular weight and a concentrated, purified protein can easily be prepared within a short period of time. On the other hand, in the conventional method, the eluent used is a polymer and the molecular weight thereof is thus almost similar to that of the protein to be isolated. Accordingly, it is difficult to separate the polymer from the protein and this in turn requires the use of a complicated treatment such as ion exchange or desalting. The method of the present invention permits the concentration and purification of a protein to not less than 3 times or to not less than 10 times, depending on conditions, by only a single run and the present invention thus permits the effective concentration and purification of a protein.

The method for concentrating and purifying a protein according to the present invention can thus easily and quite highly concentrate and purify a protein. For instance, the recovery of a protein is at best less than 50% for the usual recovery method, while it is not less than 70%, preferably not less than 75%, preferably not less than 80%, more preferably not less than 85%, particularly preferably not less than 90% and most preferably not less than 95% for the method of the present invention and the latter method can ensure an extremely high yield and is industrially and economically preferred.

Moreover, if using, as an indication, an electrical conductance (ms/cm), which corresponds to the purity as expressed in terms of the activity as an indication, the electrical conductance (ms/cm) of the resulting concentrated and purified protein is not more than 5, preferably not more than 4, more preferably not more than 3, particularly preferably not more than 2.5 and most preferably not more than 2. This clearly indicates that the content of impurities harmful to the activity of the protein is very low and the purity as expressed in terms of the activity is also high. Thus, the resulting protein composition is excellent in the storage stability and functions and is also excellent in the quality as a product.

The term "electrical conduction" herein used means such a phenomenon that charges undergo movement under the influence of an electric field to thus induce an electric current. In the mechanism of the electrical conduction, ions serve as carriers for transporting electric charges in a substance and the electrical conductance is defined to be the reciprocal of the electric resistance. The metal salt in general has a high electrical conductance and the electrical conductance serves as an indication of the amount of the remaining metal salt in a solution. The presence of metal salts adversely affects the activity and stability of the protein and therefore, the electrical conductance serves as an indication of the purity from the standpoint of the activity and that of the stability of the protein. For this reason, a higher electric resistance may adversely affect the commercial value of the resulting product.

Moreover, if using the specific activity as an indication, the method of the present invention permits the concentration of a protein to a concentration rate preferably ranging from 3 to 50 times, more preferably 5 to 50 times, further preferably 7 to 50 times and particularly preferably 10 to 50 times.

Moreover, the method of the present invention also permits the easy removal of impurities, while the protein is, for instance, in the state adsorbed on a clay mineral-containing composition and if investigating the degree of concentration and purification using the specific activity as an indication in case of, for instance, an enzyme protein, the resulting enzyme protein has functions several times to several tens of times higher than those observed for the enzymes commonly put on the market and therefore, it is confirmed that the resulting enzyme protein has improved functions and that the specific activity thereof or the concentration rate is increased to several times to several tens of times. The concentration rate has already been defined above.

In case of lipid-related enzymes such as lipase, phospholipase and cholesterol esterase, for instance, the specific activities of commercially available enzymes are on the order of about 100 U/mg, while that of the enzyme obtained after the concentration and purification according to the present invention is not less than 1000 U/mg. In other words, it is confirmed that the specific activity of the latter is not less than 10 times that of the former. This was also proved in the following Examples.

As has been discussed above, the protein composition obtained by the method of the present invention is improved in the functions, is preferred because of such highly improved functions and the commercial value of the composition is thus improved. More specifically, this means that the concentrated and purified enzyme protein or the like permits the completion of an enzyme reaction within 1/3 to 1/6 time that required for the commercially available one if the amounts of these enzymes used are identical to one another and that the concentrated and purified enzyme may ensure the same degrees of functions in an amount of 1/3 to 1/6 time that required for the commercially available one. When using the concentrated and purified enzyme in the processes for the manufacture of foods and beverages, the concentrated and purified enzyme is quite suitable since it is excellent in the productivity and production cost.

Moreover, the concentrated and purified protein may further comprise a variety of components for the improvement of, for instance, storability and stability. For instance, the protein can be improved in the stability by incorporating, into the protein, at least one member selected from the group consisting of, for instance, water-soluble polymeric compounds, divalent metal ions, antioxidants and proteins. For instance, such a water-soluble polymeric compound is preferably a polyhydric compound among others and specific examples thereof are glucose, sucrose, glycerol, sorbitol, polyvinyl alcohol and polyethylene glycol, which may be used alone or in any combination.

In addition, such divalent metal ions are, for instance, those of metals such as calcium, magnesium and copper, which may be used alone or in any combination. Moreover, such an antioxidant is, for instance, ascorbic acid, 2-mercaptoethanol, cysteine and dithiothreitol, which may be used alone or in any combination. Examples of proteins are those contained in oilseeds such as soybean, rapeseeds and sesame seed and hydrolyzates of such oilseed proteins, which may be used alone or in any combination.

Moreover, the present invention also relates to a concentrated and purified protein obtained by the foregoing concentration and purification methods, preferably a concentrated and purified enzyme protein and/or a concentrated and purified physiologically active protein and further relates to a concentrated and purified lipid-related enzyme protein.

In addition, the present invention likewise relates to a method of using these proteins in a variety of fields, in particular, in processes for manufacturing foods and beverages.

For instance, a commercially available bacterial amylase is added to starch milk (having a concentration ranging from 35 to 50%) in an amount of 0.15% with respect to that of the starch with uniformly stirring and the resulting mixture is poured and dispersed in hot water heated to a temperature ranging from 80 to 88°C. At this stage, the starch begins to gelatinize and get swollen and then liquefied due to the action of the amylase. In this case, the use of the foregoing purified amylase would permit the improvement of the heat resistance and the improvement of the rate of reaction.

Regarding the actual production processes, for instance, fattyacid-productionprocesses, a lipase is added to fats and oils in an amount ranging from 0.02 to 0.03% on the basis of the mass of the fats and oils, the same volumes of water and fats and oils are then added thereto and the reaction is continued over about 20 hours at a temperature ranging from 30 to 40°C to thus give an intended fatty acid. Although the protein in general undergoes thermal denaturation during the reaction, the lipase prepared according to the method for preparing a concentrated and purified protein according to the present invention has a high heat resistance and thus the rate of reaction is increased to a level of 1.2 to 1.3 times that expected for the commercially available enzyme or the enzyme free of any treatment of the present invention.

In case of a fat as a substitute for cacao, for instance, when producing such a fat by the transesterification between palm oil and rapeseed oil using a lipase, these oils should be reacted at a high temperature because of the high melting point of the palm oil. Contrary to this, the lipase prepared according to the method for preparing a concentrated and purified protein according to the present invention has a high heat resistance and therefore, the rate of transesterification is increased to a level of 1.1 to 1.2 times that observed for the commercial lipase.

The method of the present invention also permits the suitable treatment of a protein solution having a low concentration to give a concentrated and purified protein. In particular, the method of the invention is suitably applied to, for instance, the concentration and purification of an enzyme protein-containing culture medium to give a concentrated and purified enzyme protein. In this case, the concentration efficiency has a great influence on the production cost and therefore, it is quite important to use an enzyme having a high activity.

The method of the present invention is a particularly preferred one for concentrating and purifying enzyme proteins and physiologically active proteins since the method permits the concentration and purification of proteins to a higher degree without impairing any function thereof.

According to the method for preparing a concentrated and purified protein, a target protein can once be adsorbed on a clay mineral-containing composition and then the target protein is eluted with a small amount of an eluent. Accordingly, it has been found that the method has such a merit that theresultingelutecanefficientlybeconcentrated, purified and/or powdered without any pre-treatment or after optionally concentrating and purifying the same by the ultrafiltration.

The present invention likewise relates to a method for recovering a protein comprising the following steps (I) and (II) and, in particular, to a protein-recovery method characterized in that the protein is recovered during and/or after an enzyme reaction or a treatment with an enzyme or a method for recovering an enzyme protein from waste liquor generated in a variety of manufacturing processes and these methods are advantageous mainly from the industrial standpoint:
(I) A step for adsorbing a protein on a clay mineral-containing composition; and
(II) A step for isolating the protein adsorbed on the clay mineral-containing composition through the use of at least one member selected from the group consisting of fatty acid esters, whose fatty acid residue has a carbon atom number of not more than 30.

In particular, the foregoing method of the present invention permits the favorable recovery of, for instance, proteins such as enzyme proteins used in a variety of manufacturing processes and those, which cannot easily be recovered by the usual recovery methods. The proteins such as enzyme proteins used in such manufacturing processes are quite expensive and the effective recovery thereof would greatly have an influence on the production cost. Moreover, the foregoing method permits the recovery of proteins, which cannot easily be recovered by the usual recovery methods and therefore, the method can ensure the production of an intended product in an increased yield and the effective saving of the production cost. In particular, if recovering, for instance, a physiologically active protein, the effects of the foregoing method would further be improved. As has been described above, this method is preferred from the industrial and economical standpoints.

In this connection, the proteins obtained in, for instance, the concentration and purification methods and the recovery method according to the present invention may further be subjected to the usual concentration and purification treatments for proteins. More specifically, the concentrated and purified proteins processed by the methods of the present invention can further be concentrated using amembraneforultrafiltrationand/oranevaporator. The membrane for ultrafiltration usable herein is one whose molecular weight cutoff ranges from 6,000 to 100, 000 and the materials therefor usable herein are both polysulfone and acetonitrile type ones. The pressure and flow rate used for the ultrafiltration may appropriately and arbitrarily be changed depending on the intended proteins, but the pressure preferably ranges from 0.01 to 0.2 MPa for the inlet pressure and 0.02 to 0.3 MPa for the delivery pressure. More preferably, the inlet pressure ranges from 0.05 to 0. 12 MPa and the delivery pressure ranges from 0.08 to 0.13 MPa. Further, the concentrated and purified proteins obtained in the method of the present invention can likewise be powdered according to the usual techniques such as spray drying, lyophilization and solvent precipitation techniques. The conditions for spray drying may appropriately and arbitrarily be changed depending on the kinds and concentrations of the proteins, but the temperature preferably ranges from 50 to 150°C for the inlet temperature and 50 to 120°C for the outlet temperature and more preferably these temperatures range from 90 to 140°C and 60 to 90°C, respectively. In the lyophilization, a frozen sample is used, the pressure is controlled to a level of not more than 0.5 Toll and the temperature of the sample is desirably adjusted to the range of from 10 to 60°C , preferably 15 to 50°C and more preferably 25 to 45°C. Examples of solvents used in the solvent precipitation technique include acetone, alcohols such as methanol, ethanol and propanol, and hexane. In the solvent precipitation method, the solvent is added to the concentrated protein solution in an amount ranging from about 1 to 10 times, preferably 1 to 5 times and more preferably 2 to 3 times the volume of the protein solution to thus precipitate the protein, followed by the recovery thereof through filtration and then drying the recovered precipitates in a vacuum to thus give a powdered protein. The vacuum drying is conducted at ordinary temperature under a pressure of preferably not more than 1.0 Toll, more preferably 0.5 Toll and most preferably 0.1 Toll and the concentrated protein solution may be warmed during the drying procedure.

### [ Examples]

The present invention will hereunder be described in more detail with reference to the following Examples. In this respect, however, it is a matter of course that the scope of the present invention is not restricted to these specific Examples at all.

### Example 1

A method for eluting an enzyme protein adsorbed on and fixed to a clay mineral-containing composition and the rate of elution were investigated in this example. As the protein, a commercially available lipase (Lipase AP available from AMANO Pharmaceutical Co., Ltd.) was dissolved in ion-exchange water to a concentration of 1% and the resulting crude solution was used in this Example.

To this enzyme solution, there was added each of the clay mineral-containing compositions detailed in the following Tables 1 and 2 and whose average particle size had been adjusted to the range of from 0.1 to 10 µm in an amount of 0.2 g per 10 ml of the enzyme solution, followed by stirring the resultingmixtureover30minutesandcentrifugation at 8, 000 rpm for 10 minutes to recover not less than 95% of the clay mineral-containing composition. The clay mineral-containing composition thus recovered was washed with 10 volumes of water and then centrifuged under the same conditions used above to recover the composition. Then 10 ml each of the eluents listed in the following Table 1 was added to and dispersed in the composition recovered above, the resulting dispersion was centrifuged under the same conditions immediately after the preparation of the dispersion to give a supernatant. The eluent listed in Table 2 as Comparative Example or 10 mL of a 1.0% solution of a polymeric compound was added to the clay mineral-containing composition, the resulting mixture was stirred at room temperature for 30 minutes and then centrifuged to separate and recover the composition. The resulting supernatant was inspected for the lipase-hydrolysis activity and the mass of the protein present therein according to the Lowry method and further inspected for the rate of elution and specific activity. The results thus obtained are summarized in Table 1 and those of Comparative Example are listed in Table 2. The enzyme activity unit of lipase was defined to be the amount thereof required for releasing a fatty acid from olive oil as a substrate in an amount of 1µmol within one minute. The method for determining the hydrolysis activity of lipase used herein comprises the steps of accurately weighing out 5 mL of an emulsion of olive oil and 4 mL of a 0.1 M phosphate buffer solution (pH 7.0), thoroughly admixing them, pre-heating the resulting mixture for 10 minutes using a thermostatically controlled water bath maintained at 37°C, adding 1 mL of each sample to the mixture with stirring, reacting the mixture for 20 minutes, adding 20 mL of a mixed acetone-ethanol liquid, adding 5 drops of phenolphthalein as an indicator and then titrating the reaction system with a 0.05 N sodium hydroxide solution.

**[Table 1]**

| | | | |
|---|---|---|---|
| Table 1: Elution of enzyme proteins from clay mineral-containing compositions using a variety of fatty acid ester whose fatty acid moiety has a carbon atom number of not more than 30 | | | |

| Clay Mineral Comp. | Eluent, 0.2% aq. sol. | Recov ery (%) | Sp. Act. (U/mg) |
|---|---|---|---|
| Bentonite | Sorbitan trioleate (C60H108O8; MW: 626) | 85 | 1100 |
| | Sorbitan monostearate (C24H46O6; MW: 430) | 84 | 1300 |
| | Sorbitan monolaurate (C18H34O6; MW: 346) | 80 | 1200 |
| | Decaglycerin lauric acid ester (C43H75O16; MW: 847) | 83 | 1280 |
| | Decaglycerin stearic acid ester (C49H88O16; MW: 932) | 80 | 1100 |
| | Phosphatidyl choline (MW: 532) | 85 | 1600 |
| | Lyso-phosphatidyl ethanolamine (MW: 697) | 92 | 1700 |
| | Glycerol monostearate (C22H42O5; MW: 386) | 95 | 1800 |
| | Sucrosemonolauricacidester (C24H44O12; MW: 524) | 97 | 1960 |
| Activated clay | Sorbitan trioleate (C60H108O8; MW: 626) | 81 | 1180 |
| Montmoril lonite | Decaglycerin stearic acid ester (C49H88O16; MW: 932) | 94 | 1670 |
| Commercia 11y Enzyme liquid | -- | | 110 |
| MW: average molecular weight. | | | |

**[ Table 2]**

| | | | |
|---|---|---|---|
| Table 2: Comparative Example (Elution of an enzyme protein from clay mineral- containing compositions with a polymeric compound) | | | |
| Clay mineral comp. | Eluent | Recovery oflipase act. (%) | Sp. Act. (U/mg) |
| Bentonite | 1.0% PEG2000 | 60 | 1080 |
| | 1.0% PVA2000 | 48 | 980 |

The data listed in Table 1 indicate that the use of fatty acid esters whose fatty acid moiety has a carbon atom number of not more than 30 as eluents, according to the present invention, permits the quite efficient elution of an enzyme protein from a variety of clay mineral-containing compositions, that the degrees of concentration and purification are improved and that the specific activity of the recovered enzyme protein is improved to not less than 10 times that observed for the initial fermentation liquid per se.

On the other hand, when using the eluent shown in Table 2 as Comparative Example or an aqueous solution of a polymeric compound having a high concentration, it takes a long period of time for the elution of an intended protein. Nevertheless, the recovery is low and the specific activity of the protein thus recovered is not satisfactorily improved.

### Example 2

A commercially available powdered lipase (Lipase available from Biochemical Industries, Ltd.) was dissolved in water in such an amount that the concentration thereof was equal to 1% to give one liter of a lipase enzyme solution. To this enzyme solution, there was added 40 g of bentonite (available from WAKO Pure Chemical Co., Ltd.), the resulting mixture was stirred at 200 rpm for 10 minutes to give a dispersion and then the dispersion was centrifuged at 8, 000 rpm for 10 minutes to thus recover the bentonite. The recovered bentonite was dispersed in one liter of water, the resulting dispersion was centrifuged under the same conditions to separate and recover the bentonite and it was washed. The washed bentonite was dispersed in one liter of a 0.2% glycerin fatty acid ester solution (trade name: REODOR MS-165 available from Kao Corporation) and then the resulting dispersion was centrifuged under the same conditions to obtain a supernatant. The supernatant was inspected for the lipase activity and the amount of the protein present therein. The results thus obtained are summarized in the following Table 3.

**[ Table 3]**

| | | | | | |
|---|---|---|---|---|---|
| Table 3: Results obtained in the concentration and purification of an enzyme protein. | | | | | |

| Protein sol. | | Lipase act. (U/mL) | Recov ery (%) | Amt. Of Protein (mg/mL) | Sp. Act. (U/mg) |
|---|---|---|---|---|---|
| Clay mineral comp. | Bef. treatment | 2600 | | 15.6 | 166 |
| | Aft. treatment | 2470 | 95 | 2.0 | 1235 |

The data listed in Table 3 indicate that the adsorption, separation and elution treatments using bentonite permit the concentration and purification of the enzyme protein to a specific activity per unit mass of the enzyme of 7.4 times that observed before the treatments.

### Example 3

A commercially available lipase (Powdered Lipase QL available from Meito Sangyo Co., Ltd.) was dissolved in water to a concentration of 1% to give one liter of a lipase enzyme solution. To the resulting solution, there was added 40 g of bentonite (available from HOJUN Co., Ltd.), the resulting mixture was stirred at 200 rpm for 10 minutes to give a dispersion, the dispersion was centrifuged at 8,000 rpm for 10 minutes, the bentonite thus recovered was dispersed in one liter of water, the dispersionwascentrifugedunderthesameconditions previously used and then the bentonite recovered was washed. The bentonite thus washed was dispersed in one liter of a 0.4% sucrose oleic acid ester solution (trade name: RYOTO SUGAR ESTER S1670, available from Mitsubishi Chemical Co., Ltd.) and the resulting dispersion was centrifuged under the same conditions previously used to thus obtain a supernatant. The supernatant was subjected to concentration and desalting using a UF membrane and then inspected for the lipase activity, the amount of protein present therein and the electrical conductance. The results thus obtained are summarized in the following Table 4.

**[ Table 4]**

| | | | | | |
|---|---|---|---|---|---|
| Table 4: Results obtained in the concentration and purification of an enzyme protein | | | | | |

| Protein solution | Lipase act. (U/m L) | Recovery (%) | Amt. of protein (mg/mL) | Specific act. (U/mg) | Elec . Cond . (ms/cm) |
|---|---|---|---|---|---|
| Clay mineral comp.: Bef. The treatment | 2100 | | 12.6 | 167 | 26 |
| Released sol. or elute obtained after the treatment | 2037 | 97 | 2.1 | 1019 | 12 |
| Concentrated and desalted with UF membrane | 1780 | 85 | 0.8 | 2225 | 2.7 |

The results listed in Table 4 indicate that the adsorption, separation and elution treatments using bentonite permit the concentration and purification of the enzyme protein to a specific activity of about 13 times that observed before the treatments. Moreover, the concentration and desalting treatments with a UF membrane permit the reduction of the electrical conductance of the concentrated solution to 2.7 ms/cm and the improvement of the stability of the lipase.

### Comparative Example 1

To 100 mL of water, there was dissolved 1 g of crude pancreatin (pancreas-derived lipase available from WAKO Pure Chemical Co., Ltd.) followed by centrifugation, addition of bentonite (5 g) to the resulting supernatant and stirring for 30 minutes. Then the mixture was centrifuged at 1000 g for 10 minutes to recover the bentonite, followed by addition of 100 mL of water to the recovered bentonite to give a suspension, and centrifugation to separate and recover the bentonite. To the recovered bentonite, there was added 50 mL of a 1% PEG2000 solution as a protein-elution agent, the mixture was stirred for 30 minutes and then centrifuged to give a supernatant. Moreover, the pH value of the supernatant was adjusted to 7.5 with a 0.5 N NaOH solution, loaded on a column packed with 50 g of an ion-exchange resin: DEAE Sepharose (available from Pharmacia Company) to thus adsorb the protein on the ion-exchange resin and then unabsorbed substances were eluted with 200 mL of a 0.5M phosphate buffer (pH 7.5). Further an elute was obtained using 100 mL of a 1.0M NaCl-containing 0.5N phosphate buffer (pH 7.5) as an eluent. The resulting elute was then concentrated using AIV-3010 made of polyacrylonitrile having a molecular weight cutoff of 13,000 at an inlet pressure of 0.05 MPa, a delivery pressure of 0.1 MPa and a flow rate of 15 L/m²·hr till the volume of the elute was reduced to 1/10 time the initial volume. Moreover, 200 mL of water was added to the concentrate followed by concentration till the volume thereof was reduced to 100 mL. The resulting lipase solution was inspected for the lipase activity, the amount of the protein present therein and the electrical conductance. The results thus obtained are listed in the following Table 5.

**[Table 5]**

| | | | | | |
|---|---|---|---|---|---|
| Table 5: Separation of proteins using polymeric compounds | | | | | |

| Protein sample tested | Lipase act. (U/mL) | Recovery (%) | Protein (mg/mL) | Sp. Act. (U/mg) | Elec . Cond . (ms/cm) |
|---|---|---|---|---|---|
| Bentonite, bef. the treatment | 220 | | 5 | 44 | 24 |
| Bentonite, after the treatment | 209 | 95 | 0.8 | 261 | 18 |
| DEAE Sepharose | 140 | 63.6 | 0.5 | 280 | 48 |
| After desalting with UF membrane | 70 | 31.8 | 0.3 | 233 | 2.4 |

The polymeric compound as the protein-isolation agent was removed from the concentrateafterthetreatmentwithbentoniteusing an ion-exchange resin. However, the electrical conductance of the concentrate was 48 since a metal salt was used in the elimination processes and the stability of the lipase was reduced. At this stage, the electrical conductance of the concentrate could be reduced to 2.4 ms/cm by concentration and desalting using a UF membrane to thus remove the metal salt. However, the recovery was reduced to 31.8% due to the use of the foregoing multi-stage removal method.

### Effects of the Invention

The present invention permits the simple and efficient treatment of a large amount of an intended protein in an industrial scale, without impairing the functions thereof, for instance, without reducing the enzyme activity of the protein at low cost and also permits the concentration and purification of such a protein. Since the present invention permits the simple separation of a protein adsorbed on a clay mineral- containing composition through the use of a small amount of a separation agent, the intended protein can be obtained in a very high yield, the resulting protein has a high purity with respect to the activity or the specific activity of the protein can be increased to not less than about 3 times that observed before the treatment. According to the method of the present invention, the processes for concentrating and/or purifying proteins can considerably be simplified and the resulting highly concentrated and highly functional protein can be used in foods, medicines and a variety of manufacturing processes.

## Claims

1. A method for isolating a protein **characterized in that** a protein adsorbed on a clay mineral-containing composition is isolated using at least one member selected from the group consisting of fatty acid esters whose fatty acid residue has a carbon atom number of not more than 30.

2. The method for isolating a protein as set forth in claim 1, wherein the clay mineral-containing composition is at least one member selected from the group consisting of bentonite, montmorillonite, kaolinite, illite, chlorite, hydrotalcite, and burned and modified products thereof.

3. The method for isolating a protein as set forth in claim 1 or 2, wherein the at least one member selected from the group consisting of fatty acid esters, whose fatty acid residue has a carbon atom number of not more than 30, has an average molecular weight of less than 1000.

4. The method for isolating a protein as set forth in any one of claims 1 to 3, wherein the at least one member selected from the group consisting of fatty acid esters, whose fatty acid residue has a carbon atom number of not more than 30, comprises not less than 50% by mass of a fatty acid monoester on the basis of the total mass of the fatty acid ester.

5. The method for isolating a protein as set forth in any one of claims 1 to 4, wherein the protein is at least one member selected from the group consisting of enzyme proteins and physiologically active proteins.

6. The method for isolating a protein as set forth in any one of claims 1 to 5, wherein the protein is at least one member selected from the group consisting of lipid-related enzyme proteins.

7. The method for isolating a protein as set forth in any one of claims 1 to 6, wherein the isolation process is conducted in the presence of moisture.

8. A method for preparing a concentrated and purified protein comprising the following steps (I) and (II):
(I) A step for adsorbing a protein on a clay mineral-containing composition; and
(II) A step for isolating the protein adsorbed on the clay mineral-containing composition through the use of at least one member selected from the group consisting of fatty acid esters, whose fatty acid residue has a carbon atom number of not more than 30.

9. The method for preparing a concentrated and purified protein as set forth in claim 8, wherein it further comprises the following steps (I-A) and/or (I-B) after the completion of the foregoing step (I):
(I-A) A step for recovering the clay mineral-containingcompositiononwhichtheprotein has been adsorbed;
(I-B) A step for purifying the clay mineral-containingcompositionon which the protein has been adsorbed.

10. The method for preparing a concentrated and purified protein as set forth in claim 8 or 9, wherein the following step (III) is conducted after the completion of the foregoing step (II):
(III) A step for removing the fatty acid ester used for the isolation of the protein and/or a step for recovering the protein thus isolated.

11. The method for preparing a concentrated and purified protein as set forth in any one of claims 8 to 10, wherein the clay mineral-containing composition is added to a solution containing the protein and the resulting mixture is stirred to thus adsorb the protein on the composition; the clay mineral-containing composition carrying the protein adsorbed thereon is recovered by the use of a centrifuge or a membrane; and then the recovered clay mineral-containing composition carrying the protein adsorbed thereon is treated with at least one member selected from the group consisting of fatty acid esters, whose fatty acid residue has a carbon atom number of not more than 30, to thus isolate the protein.

12. The method for preparing a concentrated and purified protein as set forth in any one of claims 8 to 10, wherein a solution containing the protein is passed through a membrane and/or a column provided thereon with the clay mineral- containing composition to thus adsorb the protein on the mineral composition and then a solution of at least one member selected from the group consisting of fatty acid esters, whose fatty acid residue has a carbon atom number of not more than 30, is passed through the membrane and/or the column to thus isolate the protein.

13. The method for preparing a concentrated and purified protein as set forth in any one of claims 8 to 12, wherein the clay mineral-containing composition is at least one member selected from the group consisting of bentonite, montmorillonite, kaolinite, illite, chlorite, hydrotalcite, and burned and modified products thereof.

14. The method for preparing a concentrated and purified protein as set forth in any one of claims 8 to 13, wherein the clay mineral-containing composition used for adsorbing the protein has an average particle size ranging from 0.1 to 100 µm.

15. The method for preparing a concentrated and purified protein as set forth in any one of claims 8 to 14, wherein the at least one member selected from the group consisting of fatty acid esters, whose fatty acid residue has a carbon atom number of not more than 30, has an average molecular weight of less than 1000.

16. The method for preparing a concentrated and purified protein as set forth in any one of claims 8 to 15, wherein the at least one member selected from the group consisting of fatty acid esters, whose fatty acid residue has a carbon atom number of not more than 30, comprises not less than 50% by mass of a fatty acid monoester on the basis of the total mass of the fatty acid ester.

17. The method for preparing a concentrated and purified protein as set forth in any one of claims 8 to 16, wherein the protein is at least one member selected from the group consisting of enzyme proteins and physiologically active proteins.

18. The method for preparing a concentrated and purified protein as set forth in any one of claims 8 to 17, wherein the protein is at least one member selected from the group consisting of lipid-related enzyme proteins.

19. The method for preparing a concentrated and purified protein as set forth in any one of claims 8 to 18, wherein the isolation process is conducted in the presence of moisture.

20. The method for preparing a concentrated and purified protein as set forth in any one of claims 8 to 19, wherein the recovery of the protein is not less than 70%.

21. The method for preparing a concentrated and purified protein as set forth in any one of claims 8 to 20, wherein the electrical conductivity (ms/cm) of the resulting concentrated, purified protein is not more than 5.

22. The method for preparing a concentrated and purified protein as set forth in any one of claims 8 to 21, wherein the protein is concentrated and purified to a specific activity of three times the original one.

23. A concentrated and purified protein composition prepared according to the method as set forth in any one of claims 8 to 22.

24. The concentrated and purified protein composition as set forth in claim 23, wherein it further comprises at least one member selected from the group consisting of water-soluble polymeric compounds, divalent metal ions, antioxidants and proteins.

25. The concentrated and purified protein composition as set forth in claim 24, wherein the water-soluble polymeric compound is a polyhydric compound.

26. The concentrated and purified protein composition as set forth in claim 24, wherein the divalent metal ions are those of at least one metal selected from the group consisting of calcium, magnesium and copper.

27. The concentrated and purified protein composition as set forth in claim 24, wherein the antioxidant is at least one member selected from the group consisting of ascorbic acid, 2-mercaptoethanol, cysteine and dithiothreitol.

28. The concentrated and purified protein composition as set forth in claim 23 or 24, wherein the protein is at least one member selected from the group consisting of proteins derived from oilseeds and hydrolyzates thereof.

29. The concentrated and purified protein composition as set forth in any one of claims 23 to 28, wherein the protein is at least one member selected from the group consisting of enzyme proteins and physiologically active proteins.

30. The concentrated and purified protein composition as set forth in any one of claims 23 to 29, wherein the protein is at least one member selected from the group consisting of lipid-related enzyme proteins.

31. A method of using the concentrated and purified protein composition as set forth in any one of claims 23 to 30 in the production or treatment of a food or a beverage.

32. The method as set forth in claim 31, wherein the food or beverage is fats and oils and/or processed products thereof.

33. A protein-containing fatty acid ester composition obtained by isolating a protein adsorbed on a clay mineral-containing composition through the use of at least one member selected from the group consisting of fatty acid esters, whose fatty acid residue has a carbon atom number of not more than 30.

34. The protein-containing fatty acid ester composition as set forth in claim 33, wherein it further comprises at least one member selected from the group consisting of water-soluble polymeric compounds, divalent metal ions, antioxidants and proteins.

35. The protein-containing fatty acid ester composition as set forth in claim 33 or 34, wherein the water-soluble polymeric compound is a polyhydric compound.

36. The protein-containing fatty acid ester composition as set forth in claim 34, wherein the divalent metal ions are those of at least one metal selected from the group consisting of calcium, magnesium and copper.

37. The protein-containing fatty acid ester composition as set forth in claim 34, wherein the antioxidant is at least one member selected from the group consisting of ascorbic acid, 2-mercaptoethanol, cysteine and dithiothreitol.

38. The protein-containing fatty acid ester composition as set forth in claim 33 or 34, wherein the protein is at least one member selected from the group consisting of proteins derived from oilseeds and hydrolyzates thereof.

39. The protein-containing fatty acid ester composition as set forth in any one of claims 33 to 38, wherein the protein is at least one member selected from the group consisting of enzyme proteins and physiologically active proteins.

40. The protein-containing fatty acid ester composition as set forth in any one of claims 33 to 39, wherein the protein is at least one member selected from the group consisting of lipid-related enzyme proteins.

41. A method for recovering a protein comprising the following steps (I) and (II):
(I) A step for adsorbing a protein on a clay mineral-containing composition; and
(II) A step for isolating the protein adsorbed on the clay mineral-containing composition through the use of at least one member selected from the group consisting of fatty acid esters, whose fatty acid residue has a carbon atom number of not more than 30.

42. The method for recovering a protein as set forth in claim 41, wherein it further comprises the step of recovering the protein during and/or after the reaction or treatment of the protein.

43. The method for recovering a protein as set forth in claim 41 or 42, wherein an enzyme protein is recovered from a waste fluid.

44. A protein-isolation agent for isolating a protein adsorbed on a clay mineral-containing composition comprising at least one member selected from the group consisting of fatty acid esters, whose fatty acid residue has a carbon atom number of not less than 30.

45. The protein-isolation agent as set forth in claim 44, wherein the at least one member selected from the group consisting of fatty acid esters, whose fatty acid residue has a carbon atom number of not more than 30, has an average molecular weight of less than 1000.

46. The protein-isolation agent as set forth in claim 44 or 45, wherein the at least one member selected from the group consisting of fatty acid esters, whose fatty acid residue has a carbon atom number of not more than 30, comprises not less than 50% by mass of a fatty acid monoester on the basis of the total mass of the fatty acid ester.
